# NEUE EUROPÄISCHE PATENTSCHRIFT

(11) **EP 0 656 894 B2**
(45) Veröffentlichungstag und Bekanntmachung des Hinweises auf die Entscheidung über den Einspruch: **12.06.2002**
(45) Hinweis auf die Patenterteilung: 25.02.1998
(21) Anmeldenummer: 93919091.4
(22) Anmeldetag: 18.08.1993
(51) Int. Cl.: C07D 311/72, C07J 9/00

(54) **GEWINNUNG VON TOCOPHEROL UND STEROL**
ISOLATION OF TOCOPHEROL AND STEROL
PROCEDE D'OBTENTION DE TOCOPHEROL ET DE STEROL

(30) Priorität: 27.08.1992 DE 4228476
(43) Veröffentlichungstag der Anmeldung: 14.06.1995
(73) Patentinhaber: Cognis Deutschland GmbH & Co. KG, 40589 Düsseldorf (DE)
(72) Erfinder: JEROMIN, Lutz, D-40723 Hilden (DE); JOHANNISBAUER, Wilhelm, D-40699 Erkrath (DE); GUTSCHE, Bernhard, D-40721 Hilden (DE); JORDAN, Volkmar, D-40822 Mettmann (DE); WOGATZKI, Herbert, D-40625 Düsseldorf (DE)
(86) Internationale Anmeldenummer: EP9302207
(87) Internationale Veröffentlichungsnummer: WO9405650

(56) Entgegenhaltungen:
- EP-A- 0 333 472
- FR-A- 1 170 126
- GB-A- 2 218 989
- US-A- 3 335 154

## Beschreibung

Die Erfindung betrifft ein Verfahren zur Gewinnung von Tocopherol- und/oder Sterol-Konzentraten aus tocopherol- und/oder sterolhaltigen Gemischen von Fetten und/oder Fettderivaten über den Weg der Veresterung freier Fettsäuren mit Methanol, alkalikatalysierte Umesterung der Triglyceride mit Methanol und destillative Abtrennung der Fettsäuremethylester, bei dem man das Umesterungsgemisch bis zur Neutralisation ansäuert und den alkalischen Katalysator vor der Destillation auswäscht.

Tocopherolverbindungen sind in vielen pflanzlichen und tierischen Ölen enthalten und werden auch als Vitamin E bezeichnet. Die Bezeichnung Vitamin E bezieht sich auf die physiologische Wirkung dieser Nahrungsmittelinhaltsstoffe.

Es sind 8 natürlich vorkommende Substanzen mit Vitamin-E-Wirkung bekannt. Sie sind Derivate des 6-Chromanols und gehören zu 2 Gruppen von Verbindungen. Die erste Gruppe leitet sich vom Tocol ab und trägt eine gesättigte isoprenoide Seitenkette mit 16 C-Atomen. Zu dieser Gruppe gehören alpha-, beta-, gamma- und delta-Tocopherol. Die Verbindungen unterscheiden sich im Methylierungsgrad am Benzolkern des Tocols. Alpha-Tocopherol ist dabei die Substanz mit der stärksten biologischen Vitamin-E-Wirkung und der größten technischen und wirtschaftlichen Bedeutung. Es ist das dominierende Tocopherol im menschlichen und tierischen Gewebe.

Die zweite Gruppe von Substanzen mit Vitamin-E-Wirkung sind die Derivate des Tocotrienols. Sie unterscheiden sich von den anderen Tocopherol-Homologen durch die ungesättigte isoprenoide Seitenkette mit 16 C-Atomen. Die natürlich vorkommenden Toco-Enole zeigen ebenfalls eine Vitamin-E-Wirkung und werden üblicherweise zusammen mit den gesättigten Tocopherol-Homologen aus ihren natürlichen Quellen bei der Gewinnung von Vitamin E isoliert. Die Bezeichnung "Tocopherol" soll in dieser Anmeldung auch diese Tocopherol-Homologen, also alle Substanzen mit Vitamin-E-Wirkung, umfassen.

Die Tocopherole finden wegen ihreroxidationshemmenden Eigenschaften Anwendung im Nahrungsmittelund im kosmetisch-pharmazeutischen Bereich sowie als Zusatz in auf natürlichen Ölen basierenden Anstrichfarben.

Die Bezeichnung "Sterol" umfaßt in dieser Anmeldung die Sterole, die auch Sterine genannt werden. Beide Bezeichnungen "Sterol" und "Sterin" werden in dieser Patentanmeldung als gleichbedeutend benutzt. Die Sterole sind 1 -wertige sekundäre Steroidalkohole mit 27 bis 30 Kohlenstoffatomen, die die Grundstruktur des Gonans besitzen. Das Kohlenstoffatom 3 des Gonans trägt die Hydroxylgruppe. Die strukturellen Unterschiede der einzelnen bisher in der Natur gefundenen Sterole bestehen im Auftreten von Doppelbindungen innerhalb des Ringsystems, im Eintritt von Substituenten an bevorzugten Stellen und in der Konstitution der Seitenkette, die am Kohlenstoffatom 17 des Gonans verankert ist.

Der wichtigste Vertreter der Sterole ist das Cholesterin, das frei oder verestert in tierischen Organen und Flüssigkeiten, insbesondere im Gehirn, im Rückenmark, den Nebennieren, im Lebertran und im Wollfett vorkommt. Cholesterin gehört zu den sogenannten Zoosterinen, mit denen man die in tierischen Fetten enthaltenen Sterine bezeichnet. Die pflanzlichen Sterine werden Phytosterine genannt. Die wichtigsten Vertreter sind Ergosterin, Stigmasterin, Campesterin und Sitosterin. Die Sterine oder Sterole sind wertvolle Ausgangsstoffe in der Synthese von Pharmazeutika, insbesondere von Steroidhormonen, z.B. Corticosteroiden und Gestagenen. Zum Beispiel kann Stigmasterin leicht zum Progesteron umgewandelt werden.

Die Ausgangsgemische zum Gewinnen von Tocopherol und Sterol können eine Vielzahl von pflanzlichen und tierischen Substanzen sein. Die höchsten Konzentrationen von Tocopherol finden sich in pflanzlichen Ölen wie Weizenkeim-, Mais-, Soja- und Palmkernöl. Tocopherol findet sich jedoch auch in anderen Pflanzenölen, zum Beispiel Safloröl, Erdnußöl, Baumwollkeimöl, Sonnenblumenöl, Rapsöl, Palmöl und anderen Pflanzenölen.

Die natürlichen Pflanzenöle enthalten nur geringe Mengen an Tocopherol. Eine Aufkonzentrierung ist für gewerbliche Anwendungen erwünscht. Verunreinigungen sollen ferner abgetrennt werden, um die antioxidierende Wirkung und die Vitamin-E-Aktivität zu verstärken. Die wichtigsten natürlichen Quellen für Tocopherol sind daher nicht die Pflanzenöle selbst, sondern die bei der Desodorierung pflanzlicher und tierischer Öle anfallenden Wasserdampfdestillate, die auch Dämpferdestillate genannt werden. Hier fallen die Tocopherole zwar konzentriert, aber gemischt mit Sterol und Sterolestern, freien Fettsäuren sowie Triglyceriden an. Besonders interessant ist das Destillat aus der Desodorierung von Sojaöl. Auf die besondere Eignung von Sojaöl als Quelle für Tocopherole wird zum Beispiel in Fat Sci. Technol., 91. Jahrgang, 1989, S. 39 bis 41, in einem Vergleich der Desodorierungsdestillate von Soja- und Rapsöl hingewiesen. Das Sojadämpferdestillat enthält etwa 10 Ma % Mischtocopherole und in gleicher Größenordnung Sterole, die in überwiegender Menge in ihrer Esterform vorliegen.

Zum Aufkonzentrieren von Tocopherol sind unterschiedliche Verfahren bekannt, nämlich Veresterung, Verseifung und fraktionierende Extraktion. So werden Tocopherol-Konzentrate nach der DE 31 26 110 A1 aus Nebenprodukten der Desodorierung von Ölen und Fetten dadurch gewonnen, daß die in diesen enthaltenen freien Fettsäuren durch Anlagerung eines Alkohols verestert oder die freien Fettsäuren aus den Destillaten abdestilliert werden, woraufhin diese Produkte einer Hydrierung und anschließend einer Lösungsmittelfraktionierung zur Extraktion der Tocopherole unterworfen werden. Aus dem gleichen Dokument ist ein anderes Verfahren zum Aufkonzentrieren von Tocopherol bekannt. Hier werden die Desodorierungsdestillate einer Umesterung mit Methanol unterworfen und die Fettsäuremethylester abdestilliert. Der Rückstand wird durch Molekulardestillation aufkonzentriert.

Ein weiteres, aus der EP 171 009 A2 bekanntes Verfahren bringt das Tocopherol-haltige Material mit einer ausreichenden Menge eines polaren organischen Lösungsmittels in Kontakt, das die Tocopherole, aber nicht die Verunreinigungen löst. Die mit Tocopherol angereicherte polare Phase wird abgetrennt und aus dieser das Tocopherol gewonnen.

Ferner ist die Abtrennung der Tocopherole durch Adsorption an basischen Anionenaustauschern bekannt. Diese Variante ist möglich, wenn das Gemisch keine oder nur wenig Fettsäuren enthält. Die Sterole, Glyceride und andere neutrale oder basische Substanzen werden nicht adsorbiert (Ullmanns Enzyklopädie der technischen Chemie, 4. Auflage, Band 23, 1984, S. 645).

Die GB 2 145 079 A beschreibt in einem Beispiel die Verwendung saurer lonenaustauscher als Katalysator zur Veresterung von in Rapsöl-Destillat enthaltenen freien Fettsäuren mit 5 Volumenteilen Methanol, bezogen auf 1 Volumenteil Desodorisierungsdestillat. Da in Methanol unlösliche Bestandteile ausfallen, wird die Veresterung in einer Wirbelschicht vorgenommen. Die Notwendigkeit der Wirbelschicht führt zu einem aufwendigen Verfahren, dessen Wirtschaftlichkeit bei großtechnischer Durchführung zweifelhaft ist.

Mit einem weiteren, aus EP 333472 A2 bekannten Verfahren zum Herstellen hochkonzentrierter Produkte mit Tocopherol und Tocotrienol aus Palmöldämpferdestillat ist nur die Gewinnung von Tocopherol, nicht aber die von Sterol möglich. Für die Umesterung von Sterolestem sind nämlich aufgrund der relativ niedrigen Reaktionsgeschwindigkeiten Reaktionszeiten von etwa 2 Stunden erforderlich, die mit den in diesem Verfahren und zur Umesterung von Glyceriden ausreichenden Reaktionszeiten von 10 Minuten und mehr nicht erreicht werden.

Außerdem ist die fraktionierte Kristallisation zur Abtrennung der Sterole von den Tocopherolen nach der Aufkonzentrierung bekannt. Dabei geht Tocopherol in Lösung und Sterol kristallisiert aus. Auch eine destillative Trennung von Tocopherol und Sterol ist möglich, aber dabei wird Sterol, zumindest zum Teil, zerstört.

Die bekannten Verfahren zum Gewinnen von Tocopherol und Sterol haben unterschiedliche Nachteile.

Die Extraktionsverfahren müssen oft auf das Ausgangsgemisch abgestimmt werden, da die darin enthaltenen Begleitstoffe die Extraktion stark beeinflussen und die gewünschten Wertprodukte Tocopherol und Sterol bei gleichem Extraktionsverfahren und unterschiedlichen Ausgangsgemischen nicht immer in die gewünschte Phase gehen. Die bekannten Extraktionsverfahren verwenden außerdem gesundheitlich bedenkliche Lösungsmittel.

lonenaustauscher wirken spezifisch auf das Ausgangsmaterial, erfordern eine gute Vorreinigung des Gemisches und ermöglichen keine simultane Aufkonzentrierung von Tocopherol und Sterol.

Tocopherol wird in einer in dem Dokument DE 31 26 110 A1 beschriebenen Variante nach einer Veresterung der freien Fettsäuren mittels mehrwertiger Alkohole einer Molekulardestillation oder einer Wasserdampfdestillation unterworfen, um ein Destillat mit einem hohen Tocopherolgehalt zu erhalten. Der Verfahrensschritt der Molekulardestillation ist jedoch im technischen Maßstab unwirtschaftlich, und die Wasserdampfdestillation führt zu einer höheren thermischen Belastung, die die Sterole zumindest teilweise zerstört. Im letzteren Fall kann also nur das thermisch stabilere Tocopherol in guter Ausbeute gewonnen werden.

Aus der EP 0 333 472 ist ein Verfahren zur Herstellung von Tocopherolen und Tocotrienolen bekannt, bei dem man ausgehend von Fettstoffgemischen zunächst die in diesen enthaltenen freien Fettsäuren verestert, die Glyceride mit Methanol in Gegenwart von Katalysatoren verestert und schließlich die resultierenden Methylester abdestilliert. Im Rückstand verbleibt ein Tocophenolkonzentrat, das beispielsweise durch Kristallisation von höhersiedenden Verunreinigungen befreit wird.

Der Erfindung liegt daher die Aufgabe zugrunde, ein für viele unterschiedliche Ausgangsgemische anwendbares Verfahrens zum gleichzeitigen Gewinnen von Tocopherol und Sterol bereitzustellen, das ohne toxikologisch und ökologisch bedenkliche Lösungsmittel arbeitet, thermisch schonend vorgeht, gute Ausbeuten erreicht und in wirtschaftlicher Weise im technischen Maßstab durchführbar ist.

Diese Aufgabe wird erfindungsgemäß dadurch gelöst, daß man das Umesterungsgemisch bis zur Neutralisation ansäuert und den alkalischen Katalysator vor der Destillation auswäscht.

Die im Ausgangsgemisch enthaltenen freien Fettsäuren werden in einem ersten Schritt mit Methanol Fettsäuremethylester umgesetzt, um eine Verseifungsreaktion mit dem im nächsten Schritt eingesetzten Umesterungskatalysator auszuschließen.

Im nachfolgenden Verfahrensschritt, der Umesterung, werden die Sterol-Fettsäure-Ester zu Sterol und Fettsäuremethylester umgesetzt. Die Partial- und Triglyceride reagieren zu Glycerin und Fettsäuremethylester. Das im Gemisch enthaltene Tocopherol reagiert nicht. In manchen Fällen liegen nicht nur Tocopherole, sondern auch Tocopherol-Ester im Ausgangsgemisch vor, zum Beispiel im Sojaöl-Dämpferdestillat mit 0,5 Ma %. Die Ester werden in diesem Schritt in Tocopherole umgesetzt. Für den nächsten Verfahrensschritt, die Abdestillation des überschüssigen niederen Alkohols, ist es besonders vorteilhaft, wenn in den vorangegangenen Stufen ein möglichst kurzkettiger Alkohol eingesetzt worden ist, insbesondere Methanol. Auf diese Weise läßt sich die thermische Belastung niedrig halten. Wesentlich ist, dass vor der Abdestillation der Fettsäurealkylester neben der Abtrennung des Glycerins, das in der Umesterungsstufe aus gegebenenfalls vorhandenen Triglyceriden entstanden ist, auch die Entfernung des Umesterungskatalysators erfolgt. Der Katalysator liegt im wesentlichen in Form von Alkaliseife vor, die bei der Destillation stören und zum Beispiel zur Erhöhung des Siedepunktes führen könnte. Nach der Abtrennung der Fettsäurealkylester erhält man ein hochaufkonzentriertes Tocopherol-Sterol-Gemisch, aus dem Tocopherol und Sterol voneinander in an sich bekannter Weise, zum Beispiel durch Kristallisation getrennt werden können.

Ein großer Vorteil des erfindungsgemäßen Verfahrens liegt in der Vielseitigkeit der Anwendung auf unterschiedliche, Tocopherol und Sterol enthaltende Gemische. Insbesondere ist es vorteilhaft, wenn man von Sojaöl-Dämpferdestillat ausgeht. Dieses wird durch Wasserdampfdestillation des rohen Sojaöls als erste Stufe des Desodoriervorganges gewonnen. Das Destillat enthält etwa 20 % Sterol, 8 % Tocopherol, 20 % freie Fettsäuren und als Hauptbestandteil Triglyceride (Ullmann, a.a.0.).

Aber auch Dämpferdestillate anderer Öle lassen sich mit dem Verfahren verarbeiten, zum Beispiel Rapsöl-Destillate.

Das Verfahren ist jedoch nicht auf Dämpfer-Destillate pflanzlicher Öle und Fette beschränkt. Es läßt sich auch vorteilhaft auf Tallöl anwenden. Tallöl ist eines der wirtschaftlich wichtigsten Nebenprodukte des Cellulose-Sulfat-Verfahrens bei der Papierherstellung. Es wird durch Ansäuern des bei diesem Verfahren anfallenden Natriumsalz-Gemisches von Harz- und Fettsäuren erhalten. Tallöl ist ein natürliches Gemisch aus Harzsäuren vom Typ der Abietinsäure, gesättigten und ungesättigten Fettsäuren sowie Fettsäureestem und Unverseifbarem. Das Unverseifbare enthält neben höheren Alkoholen und Kohlenwasserstoffen auch Sterole.

Auch andere, Tocopherol-enthaltende Gemische lassen sich mit dem erfindungsgemäßen Verfahren aufarbeiten, zum Beispiel der bei der Rapsöl-Methylester-Herstellung anfallende Rückstand, der ebenfalls Sterole und Sterolester enthält.

In einer bevorzugten Ausgestaltung des erfindungsgemäßen Verfahrens verestert man die Fettsäuren in Gegenwart eines, insbesondere in einem Festbettreaktor vorliegenden, stark sauren lonenaustauschers bei Temperaturen von 60 bis 100° C, insbesondere von 65 bis 70° C. Vorteilhaft und überraschend war der deutlich geringere Verlust von Tocopherol durch die Löslichkeit in Methanol als bei einerdestillativen Abtrennung der Fettsäuren. Bei der Veresterung der Fettsäuren liegt das Verhältnis der Volumenströme zwischen Dämpferdestillat und niederem Alkohol zwischen 1,1 und 1,7 und vorzugsweise bei 1,4. Die Verweilzeit im Festbettreaktor beträgt 1 bis 2, vorzugsweise 1,6 Stunden. Diese Angabe bezieht sich auf das tatsächlich vorhandene freie Volumen. Bei der Veresterung werden an den aktiven Zentren des stark sauren lonenaustauschers die im Gemisch vorhandenen Fettsäuren zu Fettsäurealkylester umgesetzt.

Im Anschluß an die Reaktion wird der überschüssige niedere Alkohol, also in der Regel Methanol, in einem Phasenabscheider abgetrennt. Der Alkohol enthält außerdem den überwiegenden Teil des bei der Veresterung entstandenen Wassers.

Nach der Umesterung und der Entfernung des Überschußalkohols aus dem Reaktionsgemisch wird der Katalysator und das eventuell vorhandene Glycerin aus dem Gemisch entfernt. Vorher wird der Katalysator durch Ansäuern mit einer anorganischen Säure neutralisiert.

Im folgenden wird die Erfindung anhand von Ausführungsbeispielen näher erläutert, ohne daß eine Beschränkung auf diese Beispiele vorgenommen werden soll.

### Beispiele

### Beispiel 1: Veresterung der Fettsäuren

Sojadämpferdestillat mit einer Säurezahl von 70 wurde mit einem Volumenstrom von 0,094 l/h zusammen mit 0,067 l/h Methanol in eine 0,3 m lange Glassäule, aufgeschüttet mit Katalysator, nämlich mit einem stark sauren, makroporösen lonenaustauscherharz (Lewatit K 2631), gefördert. Der Durchmesser der Säule betrug 0,07 m. Das Gemisch wurde in einem Glasbehälter nach einer Verweilzeit von 1,6 h aufgefangen und dekantiert. Eine anschließende Eindampfung, um das Methanol/Wassergemisch von der Fettphase zu trennen, erfolgte unter Vakuum. Eine abschließend bestimmte Säurezahl ergab einen Wert von 1,3. Das entsprach einem Umsatz von 98 %, bei vemachlässigbarem Verlust an Tocopherol. Das Material ist somit für den nachfolgenden Umesterungsschritt entsäuert worden.

### Beispiel 2: Umesterung der Glyceride und Sterolester

Das im ersten Schritt entsäuerte Sojadämpferdestillat mit einer Säurezahl von etwa 1 wurde in einem Rührreaktor mit Methanol und dem basischen Katalysator in Kontakt gebracht. Die Reaktionstemperatur lag dabei zwischen 60 und 90°C, vorzugsweise bei 65°C. Bezogen auf das eingesetzte Sojadämpferdestillat wurden 40 - 80 % Methanol (vorzugsweise 50 - 60 %) und 0,8 - 1,5 % Katalysator (vorzugsweise 1 %) eingesetzt. Als Katalysator wurde vorzugsweise Natriummethylat verwendet. Möglich sind auch andere basische Katalysatoren, wie zum Beispiel Natrium-, Kalium und Lithiumhydroxid, etc. Die Reaktionszeit betrug bei 65° C ca. 2h. Nach der Umesterung waren die Sterolester zu mindestens 90 % und die Glyceride zu mindestens 95 % umgesetzt.

### Beispiel 3: Umesterung der Glyceride und Sterolester

2,8 kg entsäuertes Sojadämpferdestillat mit einer Säurezahl von 1,9 wurde mit 1,4 kg Methanol, in das vorher 192 g 30 %iges methanolisches Natriummethylat gelöst wurde, in Kontakt gebracht. Das Gemisch wurde unter ständigem Rühren auf 65° C erwärmt und 2 h bei dieser Temperatur gehalten. Um Tocopherolverluste zu vermeiden, wurde eine Stickstoffatmosphäre überlagert.

Das Ausgangsgemisch enthielt rund 6 % freie Sterole, nach Umesterung wurde nach Abzug des Methanolanteiles 16 % ermittelt. Der anfängliche Glyceridanteil von 25 % sank auf 1,2 % ab. 90 % der Glyceride waren Monoglyceride. Triglyceride waren nicht mehr nachweisbar.

### Beispiel 4: Entfernung des Überschußmethanols und Abtrennung von Katalysator und Glycerin

In Anschluß an die Umesterung wurde das überschüssige Methanol aus dem Reaktionsgemisch bei einer Temperatur von 90° C und 100 mbar abdestilliert.

Das entmethanolisierte Reaktionsgemich enthielt den eingesetzten Katalysator hauptsächlich in Form der Alkaliseife. Um den Katalysator aus dem Dämpferdestillat zu entfernen, wurde 2,2 kg entmethanolisiertes Sojadämpferdestillat mit 148 g 3 %iger Salzsäure angesäuert und mit 1,1 kg Wasser gewaschen. Im Dekanter wurden beide Phasen separiert.

### Beispiel 5: Nethylesterabtrennung

Nach einer Destillation des gebildeten Methylesters aus dem Produkt von Beispiel 4 wurde ein Gemisch erhalten, das als Hauptkomponenten 40 Ma % freie Sterole und 30 Ma % Tocopherole enthielt.

## Patentansprüche

1. Verfahren zur Gewinnung von Tocopherolen- und/oder Sterol-Konzentraten aus tocopherol- und/oder sterolhaltigen Gemischen von Fetten und/oder Fettderivaten über den Weg der Veresterung freier Fettsäuren mit Methanol, alkalikatalysierte Umesterung der Triglyceride mit Methanol und destillative Abtrennung der Fettsäuremethylester **dadurch gekennzeichnet, daß** man das Umesterungsgemisch bis zur Neutralisation ansäuert und den alkalischen Katalysator vor der Destillation auswäscht.

2. Verfahren nach Anspruch 1, **dadurch gekennzeichnet, daß** man als Gemische von Fetten und/oder Fettderivaten Dämpferdestillate des Sojaöls einsetzt.

3. Verfahren nach Anspruch 1, **dadurch gekennzeichnet, daß** man als Gemische von Fetten und/oder Fettderivaten den bei der Rapsölmethylester-Herstellung anfallenden Rückstand einsetzt.

## Claims

1. A process for the recovery of tocopherol and/or sterol concentrates from tocopherol- and/or sterol-containing mixtures of fats and/or fatty derivatives by esterification of free fatty acids with methanol, alkali-catalyzed transesterification of the triglycerides with methanol and removal of the fatty acid methyl esters by distillation, **characterized in that** the transesterification mixture is acidified to neutralization and the alkaline catalyst is removed by washing before distillation.

2. A process as claimed in claim 1, **characterized in that** steamer distillates of soybean oil are used as the mixtures of fats and/or fat derivatives.

3. A process as claimed in claim 1, **characterized in that** the residue accumulating in the production of rapeseed oil methyl ester is used as the mixture of fats and/or fat derivatives.

## Revendications

1. Procédé d'obtention de concentrés de tocophérols et/ou de stérols à partir de mélanges de graisses et/ou de dérivés de graisses contenant du tocophérol et/ou de dérivés de graisses contenant du tocophérol et/ou des stérols, par le biais de l'esterification d'acides gras libre avec le méthanol, transestérification catalysée par un alcali des triglycérides par du méthanol, et séparation par distillation des esters méthyliques d'acides gras
**caractérisé en ce qu'**
on acidifie le mélange de transestérification jusqu'à neutralisation et on élimine par lavage le catalyseur alcalin avant la distillation.

2. Procédé selon la revendication 1,
**caractérisé en ce que**
comme mélanges de graisses et/ou de dérivés de graisse on met en oeuvre des distillats atténuateurs d'huile de soja.

3. Procédé selon la revendication 1,
**caractérisé en ce que**
comme mélanges de graisses et/ou de dérivés de graisse on met en oeuvre le résidu qui se forme au cours de la production d'ester méthylique d'huile de colza.
